(19) Europäisches Patentamt
European Patent Office
Office européen des brevets

(11) Publication number: 0 270 056 A1

# (12) EUROPEAN PATENT APPLICATION

(21) Application number: 87117688.9

(22) Date of filing: 30.11.87

(51) Int. Cl.⁴: C12N 15/00 , C07H 21/04 , C12N 9/04

The microorganism(s) has (have) been deposited with American Type Culture Collection under number(s) ATCC 40283.

(30) Priority: 01.12.86 US 936451
12.06.87 US 61894

(43) Date of publication of application: 08.06.88 Bulletin 88/23

(84) Designated Contracting States: AT CH DE FR GB LI NL SE

(71) Applicant: NORTHWESTERN UNIVERSITY
633 Clark Street
Evanston Illinois 60208(US)

(72) Inventor: Goldberg, Erwin
2756 Central Park
Evanston, IL 60201(US)
Inventor: Millan, Jose Luis
7863 Camino Glorita
San Diego, CA(US)

(74) Representative: Patentanwälte Grünecker, Kinkeldey, Stockmair & Partner
Maximilianstrasse 58
D-8000 München 22(DE)

## (54) Human LDH-C 4cDNA sequences encoding antigenic regions.

(57) The field of this invention is protein, polypeptide, and peptide compounds prepared by recombinant DNA technics capable of stimulating antibodies to the lactate dehydrogenase enzyme of mammalian semen (LDH-$C_4$). Such compounds can be used to prepare vaccines for reducing fertility of female nammals including women.

EP 0 270 056 A1

# HUMAN LDH-C cDNA SEQUENCES ENCODING ANTIGENIC REGIONS

## FIELD OF INVENTION

The field of this invention is protein, polypeptide, and peptide compounds capable of stimulating antibodies to the lactate dehydrogenase enzyme of mammalian semen (LDH-$C_4$). Such compounds can be used to prepare vaccines for reducing fertility of female mammals including women.

## BACKGROUND OF INVENTION

Mammalian spermatozoa have been known to be antigenic for many years. More recently, it has been demonstrated that mammalian sperm contain an antigenic enzyme, which is known as the $C_4$ isoenzyme of lactate dehydrogenase (LDH-$C_4$). LDH-$C_4$ has been isolated in pure crystalline form from mouse testes. Goldberg (1972), J. Biol. Chem. 247:2044-2048. The enzyme has a molecular weight of 140,000 and is composed of four identical C subunits. The amino acid sequence and three-dimensional structure of mouse LDH-$C_4$ have been studied and described by a number of investigators: Musick, et al. (1976), J. Mol. Biol. 104:659-668; Wheat, et al. (1977), Biochem. & Biophys. Res. Comm. 74, No. 3:1066-1077; Li, et al. (1983), J. Biol. Chem. 258:7017-7028; and Pan, et al. (1983), J. Biol. Chem. 258: 7005-7016.

In 1975, Dr. Erwin Goldberg reviewed the effects of immunization with LDH-X (LDH-$C_4$) on fertility, and advanced the possibility that "by using a defined macromolecular constituent of sperm it becomes possible to elucidate its primary structure in terms of amino acid sequence, to map specifically the antigenic determinant(s) responsible for inducing infertility, and then to construct synthetic peptides containing these determinants. Possessing the capability for synthesizing a molecule with such properties makes the immunological approach to fertility control feasible". Karolinska Symposia on Research Methods in Reproductive Endocrinology 7th Symposium: Immunological Approaches to Fertility Control, Geneva, 1974, 202-222.

Subsequent investigations by Dr. Goldberg and his research associates identified several amino acid sequences of mouse LDH-$C_4$ which in isolated form (e.g., as short chain peptides) bind to LDH-$C_4$ antiserum. Wheat, et al. (1981) in Rich, et al., Peptides: Synthesis-Structure-Function, Proc. 7th Amer. Peptide Symp., pp. 557-560; and Gonzales-Prevatt, et al. (1982), Mol. Immunol. 19:1579-1585. Antigenic peptide compounds based on these sequences have been patented. See U.S. Patents Nos. 4,290,944; 4,310,456; 4,353,822; 4,377,516; 4,392,997; 4,578,219; and 4,585,587.

These antigenic peptides are useful in preparing vaccines to reduce female fertility. Immunization of female mammals results in the development of circulating antibodies specific to LDH-$C_4$. These immunoglobulins reach the female reproductive tract as a transudate of serum. Kille, et al. (1977), Biol. Reprod. 20:863-871. Antibody in cervical mucus, uterine fluids, and oviducal fluids combine with LDH-$C_4$ on the sperm surface and impede the progress of the male gamete, presumably by agglutination. Systemic immunization with LDH-$C_4$ markedly interferes with sperm transport in the female reproductive tract. Kille et al. (1980), J. Reprod. Immunol. 2:15-21.

The current status of research on LDH-$C_4$ and antigenic peptides for use in female contraceptive vaccines is summarized in two earlier publications by the Goldberg group: Goldberg, et al. (1983), In Immunology of Reproduction, Chapt. 22, pp. 493-504; and Wheat, et al. (1983) in Isozymes: Current Topics in Biological and Medical Research, Vol. 7, pp. 113-140, and most recently in Goldberg and Shelton, "Immunological Approaches to Contraception and Promotion of Fertility", pages 219-230 (ed. G. P. Talwar, Plenum Publishing Corp., 1986). In the prior work on synthetic antigenic peptides corresponding to antigenic regions of LDH-$C_4$, it was assumed that the antigenic regions of mouse LDH-$C_4$ enzyme were essentially homologous with those of human LDH-$C_4$. Tests in rabbits had demonstrated immunization effects from the mouse antigenic sequences. The synthetic peptide corresponding to the mouse LDH-$C_4$ sequence 5-15 was found to be antigenic for immunizing not only female rabbits but also female baboons.

## SUMMARY OF INVENTION

As a result of the experimental work leading to the present invention, the nucleotide sequence of the human Ldh-c gene has been determined. The cDNA encoding a subunit of the human isozyme was identified, isolated, and cloned, and has been incorporated in synthetic recombinant DNA molecules, including both phage and plasmid vectors. Since the cDNA for the complete subunit contains an internal Eco RI site, it can readily be cleaved into two segments both of which contain DNA sequences encoding antigenic regions of the isozyme. The complete cDNA contains six principal antigenic regions which can be used either separately or in combination to produce a protein or peptide containing one or more antigenic regions. The antigenic regions of human LDH-$C_4$ all differ in their amino acid sequences from the corresponding antigenic regions of mouse LDH-$C_4$. For the purpose of preparing antigenic compounds by recombinant DNA procedures, it is therefore preferred to utilize the human cDNA sequences of this invention, and thereby obtain fully human homologous antigenic regions in the corresponding proteins or peptides.

## DETAILED DESCRIPTION

Standard nomenclature and abbreviations are used for the nucleotide bases of the DNA sequences described herein: A for adenine; T for thymine; G for guanine; and C for cytosine. When one strand of double-stranded DNA is shown, such as cDNA sequences, it will be understood that complementarity specifies that adenine binds to thymine and guanine to cytosine: A-T (or T-A) and G-C (or C-G).

The cDNA sequence encoding human LDH-$C_4$ subunit represented in the following Diagram A contains 993 bases providing 33l codons which encode the 33l amino acids of the human LDH-$C_4$ subunit. Diagram A shows the chemical sequence numbers for the bases which are divided into the codons with the corresponding amino acids in paired relation. As indicated in Diagram A, there is an internal Eco RI site between the 92l guanine and 922 adenine.

The complete encoding cDNA sequence of Diagram A has been incorporated in the plasmid pGEM-4 (supplied by Promega Biotec, Madison, Wisconsin). This plasmid is identified by the designation pGEM-4+humLdh-c. That recombinant plasmid to facilitate the filing of this application has been placed on deposit with The American Type Culture Collection, l230l Parklawn Drive, Rockville, MD 20852. The humLdh-c in the plasmid has untranslated 5′ and 3′ flanking sequences as represented in the following Diagrams B and C.

The 5′ flanking region includes an Eco RI restriction site at its outer end, and there is a modified pGEM-4 polylinker at the 3′ end which contains a plurality of restriction sites, as indicated in the footnote to Diagram C. By cleaving the plasmid pGEM-4+ humLdh-c with an Eco RI restriction enzyme, an encoding sequence can be obtained including bases l to 92l. The sequences of this large fragment encode a plurality of antigenic regions. By using a further restriction enzyme, which can be any of the ones cleaving the modified pGEM-4 polylinker, a shorter fragment can be obtained including the bases 922 to 993, which include encoding sequences for one complete antigenic region and the larger part of another antigenic region. The location and correspondence of these regions will be further discussed below.

DIAGRAM A

cDNA Sequences Encoding Human LDH-$C_4$ Subunit*

```
                                                                         1
                                                                5'-ATG TCA ACT        6
                                                                   Met Ser Thr        2
                                                                       1
GTC AAG GAG CAG CTA ATT GAG AAG CTA ATT GAG GAT GAT GAA AAC TCC CAG TGT AAA ATT ACT   69
Val Lys Glu Gln Leu Ile Glu Lys Leu Ile Glu Asp Asp Glu Asn Ser Gln Cys Lys Ile Thr   23
        (5)                                     (15)
ATT GTT GGA ACT GGT GCC GTA GGC ATG GCT TGT GCT ATT AGT ATC TTA CTG AAG GAT TTG GCT   132
Ile Val Gly Thr Gly Ala Val Gly Met Ala Cys Ala Ile Ser Ile Leu Leu Lys Asp Leu Ala   44

GAT GAA CTT GCC CTT GTT GAT GTT GCA TTG GAC AAA CTG AAG GGA GAA ATG ATG GAT CTT CAG   195
Asp Glu Leu Ala Leu Val Asp Val Ala Leu Asp Lys Leu Lys Gly Glu Met Met Asp Leu Gln   65

CAT GGC AGT CTT TTC TTT AGT ACT TCA AAG GTT ACT TCT GGA AAA GAT TAC AGT GTA TCT GCA   258
His Gly Ser Leu Phe Phe Ser Thr Ser Lys Val Thr Ser Gly Lys Asp Tyr Ser Val Ser Ala   86

AAC TCC AGA ATA GTT ATT GTC ACA GCA GGT GCA AGG CAG CAG GAG GGA GAA ACT CGC CTT GCC   321
Asn Ser Arg Ile Val Ile Val Thr Ala Gly Ala Arg Gln Gln Glu Gly Glu Thr Arg Leu Ala   107

CTG GTC CAA CGT AAT GTG GCT ATA ATG AAA ATA ATC ATT CCT GCC ATA GTC CAT TAT AGT CCT   384
Leu Val Gln Arg Asn Val Ala Ile Met Lys Ile Ile Ile Pro Ala Ile Val His Tyr Ser Pro   128

GAT TGT AAA ATT CTT GTT GTT TCA AAT CCA GTG GAT ATT TTG ACA TAT ATA GTC TGG AAG ATA   447
Asp Cys Lys Ile Leu Val Val Ser Asn Pro Val Asp Ile Leu Thr Tyr Ile Val Trp Lys Ile   149

AGT GGC TTA CCT GTA ACT CGT GTA ATT GGA AGT GGT TGT AAT CTA GAC TCT GCC CGT TTC CGT   510
Ser Gly Leu Pro Val Thr Arg Val Ile Gly Ser Gly Cys Asn Leu Asp Ser Ala Arg Phe Arg   170

TAC CTA ATT GGA GAA AAG TTG GGT GTC CAC CCC ACA AGC TGC CAT GGT TGG ATT ATT GGA GAA   573
Tyr Leu Ile Gly Glu Lys Leu Gly Val His Pro Thr Ser Cys His Gly Trp Ile Ile Gly Glu   191

CAT GGT GAT TCT AGT GTG CCC TTA TGG AGT GGG GTG AAT GTT GCT GGT GTT GCT CTG AAG ACT   636
His Gly Asp Ser Ser Val Pro Leu Trp Ser Gly Val Asn Val Ala Gly Val Ala Leu Lys Thr   212
                                                                             (211)
CTG GAC CCT AAA TTA GGA ACG GAT TCA GAT AAG GAA CAC TGG AAA AAT ATC CAT AAA CAA GTT   699
Leu Asp Pro Lys Leu Gly Thr Asp Ser Asp Lys Glu His Trp Lys Asn Ile His Lys Gln Val   233
                                                    (226)               (231)
ATT CAA AGT GCC TAT GAA ATT ATC AAG CTG AAG GGG TAT ACC TCT TGG GCT ATT GGA CTG TCT   762
Ile Gln Ser Ala Tyr Glu Ile Ile Lys Leu Lys Gly Tyr Thr Ser Trp Ala Ile Gly Leu Ser   254
                                       (243)
GTG ATG GAT CTG GTA GGA TCC ATT TTG AAA AAT CTT AGG AGA GTG CAC CCA GTT TCC ACC ATG   831
Val Met Asp Leu Val Gly Ser Ile Leu Lys Asn Leu Arg Arg Val His Pro Val Ser Thr Met   277

GTT AAG GGA TTA TAT GGA ATA AAA GAA GAA CTC TTT CTC AGT ATC CCT TGT GTC TTG GGG CGG   394
Val Lys Gly Leu Tyr Gly Ile Lys Glu Glu Leu Phe Leu Ser Ile Pro Cys Val Leu Gly Arg   298
                              (233)  921\ ▼ /922
AAT GGT GTC TCA GAT GTT GTG AAA ATT AAC TTG AAT TCT GAG GAG GAG GCC CTT TTC AAG AAG   957
Asn Gly Val Ser Asp Val Val Lys Ile Asn Leu Asn Ser Glu Glu Glu Ala Leu Phe Lys Lys   319
                                            987                             (316)
AGT GCA GAA ACA CTT TGG AAT ATT CAA AAG GAT CTA ATA TTT TAA-3'                        993
Ser Ala Glu Thr Leu Trp Asn Ile Gln Lys Asp Leu Ile Phe ***                           331
                         (324)                  (330)
```

*Encoding base units chemical sequence numbers are indicated in the right-hand column 1 to 993 and the corresponding amino acids 1 to 331. These numbers do not include the ATG start or the end TAA. An internal Eco RI restriction site is indicated by symbol "▼" between bases 921 and 922.

## DIAGRAM B

Untranslated Bases of 5' Flanking Region*

```
                                                          v
                                                     5'-GAATTCGGGCGCCTCAA
CTGTCGTTGGTGTATTTTTCTGGTGTCACTTCTGTGCCTTCCTTCAAAGGTTCTCCAA ATG TCA ACT-
```

*An Eco RI restriction site is indicated by symbol "v". Three connecting codons are also shown for orientation of untranslated bases.

## DIAGRAM C

Untranslated Bases of 3' Flanking Region and Terminal Polylinker*

```
                                         -ATA TTT TAA ATTAAAGCCTTCTAATGTTCCACTGTTTGGA
GAACAGAAGATAGCAGGCTGTGTATTTTAAATTTTGAAAGTATTTTCATTGATCTTAAAAAATAAAACAAATTGGAGACCTG
AAAAAAAAAAAAAAAAGGCCACACTTTATTTTAAAGGG-polylinker-3'
```

*Modified polylinker of pGEM-3 plasmid contains Ava I, Bam HI, Xba I, Hinc II, Acc I, Sal I, Pst I, Sph I, and Hind III restriction sites. Three connecting codons are also shown for orientation of untranslated bases.

In the following comparison, standard amino acid names and abbreviations are used, as set out in the following table.

Names and Abbreviations of Amino Acids

| Amino Acids | Three-letter Abbreviations |
|---|---|
| alanine | Ala |
| arginine | Arg |
| asparagine | Asn |
| aspartic acid | Asp |
| cysteine | Cys |
| glutamine | Gln |
| glutamic acid | Glu |
| glycine | Gly |
| histidine | His |
| isoleucine | Ile |
| leucine | Leu |
| lysine | Lys |
| methionine | Met |
| phenylalanine | Phe |
| proline | Pro |
| serine | Ser |
| threonine | Thr |
| tryptophan | Trp |
| tyrosine | Tyr |
| valine | Val |

Human LDH-$C_4$ as represented by the cDNA sequences of Diagram A, differs significantly from mouse LDH-$C_4$, as reported in the literature. See Wheat, et al. (1977), Biochem. & Biophys. Res Comm. 74, No. 3:1066-1077; Li, et al. (1983), J. Biol. Che., 258:7017-7028; and Pan, et al. (1983), J. Biol. Chem. 258:7005-7016. The principal regions of the corresponding enzyme subunit differ markedly in amino acid content, as shown by the following comparison.

```
     5               9   10      12  13  14a 14b 15
(1)  Glu-Gln-Leu-Ile-Glu-Lys-Leu-Ile-Glu-Asp-Asp-Glu
                     (Gln)(Asn)  (Val)(Pro)(Glu) (Lys)

     211     213     214 215                                     226
(2)  Thr-Leu-Asp-Pro-Lys-Leu-Gly-Thr-Asp-Ser-Asp-Lys-Glu-His-Trp-Lys
     (Ser)   (Asn)   (Ala)(Ile)

     231     233 234 235 236         239 240 241 242 243
(3)  Gln-Val-Ile-Gln-Ser-Ala-Tyr-Glu-Ile-Ile-Lys-Leu-Lys
             (Val)(Glu)(Gly)(Gly)    (Val)(Leu)(Asn)(Met)

     283     285                                 295 296
(4)  Glu-Glu-Leu-Phe-Leu-Ser-Ile-Pro-Cys-Val-Leu-Gly-Arg-Asn-Gly-
             (Val)                               (Glu)(Ser)

     298 299     301     303
     Val-Ser-Asp-Val-Val-Lys
     (Ile)(Thr)  (Phe)

     304     306 307 308             312     314     316
(5)  Ile-Asn-Leu-Asn-Ser-Glu-Glu-Glu-Ala-Leu-Phe-Lys-Lys
     (Val)   (Met)(Thr)(Ala)         (Gly)   (Leu)

     324                 329 330
(6)  Ile-Gln-Lys-Asp-Leu-Ile-Phe
     (Met)               (Glu)(Leu)
```

The amino acids of the above peptides are all L-amino acids with the exception of glycine which does not have L-D forms. The numbers shown above the amino acids correspond to the numbering system previously used for amino acids of mouse LDH-$C_4$. See Wheat and Goldberg (1985), Annals N.Y. Acad. Sci., 438:156-169. Mouse LDH-$C_4$ was numbered 1 to 330 with the insertion of aspartic acid treated as follows: Pro-13, Glu-14a, Asp-14b, Lys-15, etc. The mouse sequence numbers are shown in parenthesis on Diagram A.

The amino acids indicated in parentheses under certain of the amino acids of peptides 1 to 6 represent the different amino acids of the corresponding sequences of mouse LDH-$C_4$. With reference to peptide (1), amino acids 9, 10, 12, 13, 14a and 15 are different in the human LDH-$C_4$ than in the mouse LDH-$C_4$. Similar differences are shown with respect to each of the peptide compounds, peptide (2) having four different amino acids, peptide (3) having eight different amino acids, peptide (4) having six different amino acids, peptide (5) having six different amino acids, and peptide (6) having three different amino acids.

The cDNA sequences of this invention can be utilized to prepare proteins (viz. long chain polypeptides) which are immunogenic and can be used without coupling to an antigenic protein in preparing anti-fertility vaccines. Alternatively, a fusion protein consisting of the cDNA sequence, or a portion thereof, and the beta galactosidase of the vector would be immunogenic. When the polypeptide or peptide prepared by recombinant DNA procedures from segments of the complete cDNA coding subunit are not sufficiently immunogenic because of their short length, they can be conjugated to a carrier molecule, which is preferably a protein which itself elicits an antigenic response and which can be safely administered. For example, the short chain polypeptide or peptide can be coupled to tetanus toxoid for administration by intramuscular injection. For example, a mixture of 1µMole tetanus toxoid, 60 µMoles antigenic peptide, and 18 millimoles 1-ethyl-3-(3 dimethyl aminopropyl) carbodiimide hydrochloride reacted in water (pH6) for 12 hours at room temperature and 24 hours at 4° gives a product containing 3.5 moles of peptide/mole tetanus toxoid. Excess reactants can be removed by dialysis or gel filtration. See Pique et al., Immunochemistry 15:55-60 (1978). Alternatively, the short chain polypeptide or peptide may be coupled using bisadiazotized benzidine (Bassiri et al., Endocrinology, 90-722 (1972)) or glutaraldehyde. The short chain polypeptides or peptide can also be made immunogenic by adding a fatty acid (lauric acid) or a short series of hydrophobic amino acids (Phe-Leu-Leu-Val-Val-Cys-Tyr-Gly-Gly) to one end of the peptides and hydrophobically binding that end to meningococcal outer membrane proteins (Smith, et al., 6th Int'l Congress of Immunol. Abst., p. 248, 1986; Lowell, G., personal communication to E.G., 1986).

For intramuscular injection, the subunit LDH protein, an immunogenic polypeptide or a protein-coupled peptide may be suspended in a sterile isotonic saline solution or other conventional vehicle, and, if desired, an adjuvant may be included. A preferred use of such a vaccine is for administration to human females. Antibodies will be formed which will appear in the oviduct fluids and thereby produce a significant reduction in fertility. For this purpose, the amount to be administered will range from about 1 to 10 milligrams (mg) of the antigenic peptide.

The scientific basis of the present invention as well as its practical application are more fully illustrated by the following experimental examples.

## EXPERIMENTAL EXAMPLES

### EXAMPLE I

A human testis specimen was obtained after coadjuvant orchiectomy during operation of a prostatic cancer. RNA was extracted from the testis by the guanidinium isothiocyanate procedure followed by CsCl centrifugation, and total polyadenylated RNA was selected by oligo dT-cellulose. A cDNA expression library was constructed in λ gtll following a procedure previously reported for the generation of a human placental library. Millan (l986), J. Biol. Chem., 25l:3ll2-3ll5. The cDNA larger than l.0 kb was ligated to λ gtll arms. The recombinant DNA was packaged using the Gigapack System (Vector Cloning Systems, San Diego, CA). Recombinant phages were obtained at an efficiency of 35,000 plaques/ng of double-stranded cDNA. The library, consisting of $l.8 \times l0^6$ independent recombinant phages and containing less than 5% nonrecombinants, was amplified on E. coli Yl088.

#### Immunochemical screening

Three high titer rabbit sera produced by immunization with purified mouse LDH-$C_4$ protein were pooled and used to screen the human testis cDNA expression library. The antiserum was diluted l00-fold and absorbed with a lysate of E. coli strain Yl090. The testis λ gtll library was plated at a density of $2.5 \times l0^4$ plaque-forming units (pfu) per l50 $mm^3$ petri dish with E. coli Yl090 as host bacteria. After growth at 42°C and induction with IPTG, the nitrocellulose filters were screened and bound antibody detected by use of goat anti-rabbit IgG coupled to horseradish peroxidase. The generation of murine monoclonal antibodies against mouse LDH-$C_4$ has previously been reported. Goldman-Leiken and Goldberg (l983), Proc. Nat'l. Acad. Sci. USA, 74:5463-5467. Monoclonal antibody $F_6$ $H_8$ was used in a secondary screening and binding was detected with goat antimouse IgG labeled with horseradish peroxidase.

#### Sequence Analysis

cDNA inserts were subcloned into the Eco RI site of Ml3mpl9 and sequenced using the universal l7-mer sequencing primer (P-L Biochemicals) and l7-and l8-mer oligonucleotides synthesized on an Applied Biosystems DNA synthesizer. Sequencing of the clones was accomplished by the Sanger dideoxy chain termination procedure using the Klenow fragment of DNA polymerase and 35SdATP as tracer. See Sanger et al., Proc. Nat'l. Acad. Sci. USA, 74:5463-5467; and Biggen et al. (l983), Proc. Nat'l. Acad. Sci. U.S.A., 80:3963-3965.

The pooled antisera prepared as described above reacted with l2 putative clones upon screening $l.8 \times$ l05 pfu from the expression library. The positive clones were isolated as single plaques and rescreened as macroplaques. Three of these twelve putative clones tested positive with the $F_6$ $H_8$ monoclonal antibody to mouse LDH-$C_4$. These three clones contained inserts 0.8, 0.9, and l.0 kbp long that cross-hybridized with each other in Southern blotting. The clone selected for sequence analysis contained a noncross-hybridizing fragment of 0.6 kbp in addition to the l.0 kbp cDNA suggesting an internal Eco RI restriction site in the LDH-$C_4$ cDNA. In Northern blot analysis, the nick-translated l.0 kb subfragment detected a single mRNA species of approximately l.5 kb in human testis RNA but not in placental RNA. The determined sequence of the human LDH-$C_4$ cDNA subunit is shown in Diagram A. The complete cloned cDNA included the flanking regions illustrated in Diagrams B and C. The l.0 kbp subfragment includes 66 bp of 5' flanking region and encodes most of the LDH-$C_4$ protein. The 0.6 kbp subfragment codes for the last 24 amino acids of LDH-$C_4$, a flanking region and a poly A tail.

EXAMPLE II

The plasmid pGEM-4 +humLdh-c was prepared as follows:

The human Ldh-c cDNA was cloned from a λ gtll human testis cDNA library as described in Example I. The recombinant phage containing the Ldh-c cDNA was digested with Eco RI. Two fragments sized l.0 and 0.6 kb were isolated, indicating an internal unmethylated Eco RI site. Large scale DNA preparations were carried out with the recombinant Ml3 phages. The two Ldh-c cDNA fragments were recovered from the Ml3 vectors with Eco RI digestion. The l.0 kb fragment was subcloned into the Eco RI site of plasmid vector pGEM4 (Promega Biotec, Madison, WI), and the 0.6 kb fragment was subcloned into the Eco RI site of pGEM-3 (Promega Biotec). Large quantities of DNA were then prepared from the plasmid vectors. The two fragments were subcloned together in the correct orientations to produce one fragment of human Ldh-c cDNA with a regenerated internal Eco RI site. However, first the 0.6 kb fragment was manipulated further.

The 0.6 kb fragment contained approximately 200 bp of Ldh-c cDNA up to the poly A tail, but in addition, contained 400 bp of downstream DNA from an unknown origin, believed to be an artifact of cDNA cloning. To eliminate this spurious DNA, the pGEM-3+ 0.6 kb Ldh-c DNA was digested with Fok I. The Ldh-c cDNA contains a Fok I cutting site l4-l7 bp downstream of the poly A tail. The Fok I site has a 5′ overhang which was filled in with deoxynucleotides and T4 DNA polymerase. The Fok I fragments from digestion of the recombinant plasmid were separated by agarose gel electrophoresis. The Fok I fragment containing the Ldh-c cDNA was recovered, and subsequently digested with EcoRI. This generated a 220 bp Ldh-c fragment, with the natural Eco RI site at the 5′ end, and the blunt-ended filled-in Fok I site at the 3′ end with l7 bp of unknown DNA after the poly A tail. The 220 bp fragment forms the 3′ portion of the whole Ldh-c cDNA. The l.0 kb fragment comprises the 5′ portion.

The pGEM-4 vector with the l.0 kb Ldh-c cDNA fragment was digested with Sma I to linearize the plasmid at the multi ple cloning site downstream of the insert. This was dephosphorylated. Subsequently, the DNA was partially digested with Eco RI to generate an intact l.0 kb Ldh-c insert in the pGEM-4 vector, with only the l8 bp Eco RI to Sma I piece of the multiple cloning site excised. Finally, the 5′ Eco RI, 3′ blunt-ended 220 bp Ldh-c fragment was subcloned downstream of the l.0 kb Ldh-c fragment in the Eco RI-Sma I cut recombinant pGEM4 plasmid.

The resulting plasmid has been designated "pGEM-4+ Ldh-c", and has been placed on deposit with American Type Culture Collection, Rockville, MD, 20852, U.S.A., under ATCC Accession No. 40283. This deposit has Budapest Treaty status.

The pGEM-4+humLdh-c plasmid can be stored as lyophilized DNA or resuspended DNA in l0 mM Tris, l mM EDTA. Alternatively, the plasmid can be preserved in frozen stocks of transformed E. coli cells. The recommended E. colihost strain for cloning is HBl0l.

The human Ldh-c cDNA can be isolated from the pGEM-4+humLdh-c plasmid as one l.2 kb fragment from the pGEM4+human Ldh-c plasmid by complete digestion with Hind III, followed by partial digestion with Eco RI. The l.2 kb fragment can be separated by agarose gel electrophoresis, and subsequently recovered.

EXAMPLE III

For preparation of large quantities of pGEM4+humLdh-c DNA, the procedures listed below should be followed as described in Molecular Cloning: A Laboratory Manual by Maniatis, Fritsch, and lambrook (Cold Spring Harbor, New York, l982). First, pGEM4+humLdh-c should be introduced into an appropriate E. coli host strain such as HBl0l (see "Transformation by the calcium chloride procedure"). Subsequently, the transformed bacteria can be grown up to a large volume (see "Growth of bacteria and amplification of the plasmid"), from which the plasmid DNA can be isolated (see "Harvesting and lysis of bacteria, lysis by alkali") and purified by density gradient centrifugation (see "Purification of closed circular DNA by centrifugation to equilibrium in cesium chloride-ethidium bromide gradients"). The final product will be pGEM4+humLdh-c DNA dissolved in l0 mM Tris, l mM EDTA.

The pGEM4+hum Ldh-c plasmid can be stored as lyophilized DNA, or resuspended DNA in l0 mM Tris, l mM EDTA. Alternatively, the plasmid can be preserved in frozen stocks of transformed E. coli cells.

## Claims

l. A synthetic recombinant DNA molecule containing a cDNA sequence encoding at least one antigenic region of the subunit of human testis-specific lactate dehydrogenase isozyme (human LDH-$C_4$ )

2. The synthetic recombinant DNA molecule of claim l in which said cDNA sequence encodes a plurality of the antigenic regions of human LDH-$C_4$.

3. A synthetic recombinant DNA molecule containing a cDNA sequence encoding essentially the complete subunit of human testis-specific lactate dehydrogenase isozyme (human LDH-$C_4$ ).

4. A synthetic recombinant DNA molecule containing the cDNA sequence l to 92l of Diagram A.

5. A synthetic recombinant DNA molecule containing the cDNA sequence 922 to 003 of Diagram A.

6. DNA cloning and/or expression vectors containing the cDNA encoding sequence of claims l, 2, 3, 4 or 5.

7. DNA plasmid vectors containing the cDNA encoding sequence of claims l, 2, 3, 4 or 5.

8. Lambda phage vectors containing the cDNA encoding sequence of claims l, 2, 3, 4 or 5.

9. The plasmid vector pGEM-4 + humLdh-c.

European Patent Office

# EUROPEAN SEARCH REPORT

Application number

EP 87117688.9

| Category | DOCUMENTS CONSIDERED TO BE RELEVANT<br>Citation of document with indication, where appropriate, of relevant passages | Relevant to claim | CLASSIFICATION OF THE APPLICATION (Int. Cl 4) |
|---|---|---|---|
| Y,P | CHEMICAL ABSTRACTS, vol. 107, no. 11, September 14, 1987, Columbus, Ohio, USA<br>I. SAKAI et al. "Molecular cloning and nucleotide sequence of the cDNA for sperm-specific lactate dehydrogenase-C from mouse"<br>page 192, left column, abstract no. 91 092y<br>& Biochem. J. 1987, 242(2), 619-22<br>-- | 1-3 | C 12 N 15/00<br>C 07 H 21/04<br>C 12 N 9/04 |
| D,Y | THE JOURNAL OF BIOLOGICAL CHEMISTRY, vol. 258, no. 11, June 10, 1983 (Baltimore, USA)<br>Y.-C.E. PAN et al. "Amino Acid Sequence Studies on Lactate Dehydrogenase $C_4$ Isozymes from Mouse and Rat Testes"<br>pages 7005-7016<br>* Page 7009; fig. 3 *<br>-- | 1-3 | TECHNICAL FIELDS SEARCHED (Int. Cl 4)<br>C 12 N<br>C 07 H<br>A 61 K |
| A | CHEMICAL ABSTRACTS, vol. 102, no. 17, April 29, 1985, Columbus, Ohio, USA<br>H. TSUJIBO et al. "Nucleotide Sequences of the cDNA and an intronless pseudogene for human lactate dehydrogenase-A isozyme"<br>page 140, left column, abstract no. 144 027m<br>& Eur. J. Biochem. 1985, 147(1), 9-15<br>-- | 1 | |

The present search report has been drawn up for all claims

| Place of search | Date of completion of the search | Examiner |
|---|---|---|
| VIENNA | 09-02-1988 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application number

EP 87117688.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | THE JOURNAL OF BIOLOGICAL CHEMISTRY, vol. 258, no. 11, June 10, 1983 (Baltimore, USA) S.S.-L. LI et al. "Molecular Features and Immunological Properties of Lactate Dehydrogenase $C_4$ Isozymes from Mouse and Rat Testes" pages 7017-7028 * Totality * -- | 1 | |
| D,A | MOLECULAR IMMUNOLOGY, vol. 19, no. 12, December 1982 (Oxford, GB) V. GONZALES-PREVATT et al. "Identification of an Antigenic Determinant of Mouse Lactate Dehydrogenase $C_4$" pages 1579-1585 * Totality * -- | 1 | TECHNICAL FIELDS SEARCHED (Int. Cl.4) |
| D,A | US - A - 4 585 587 (GOLDBERG et al.) * Totality * ---- | 1 | |

The present search report has been drawn up for all claims

| Place of search | Date of completion of the search | Examiner |
|---|---|---|
| VIENNA | 09-02-1988 | WOLF |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document